# EUROPEAN PATENT SPECIFICATION

(11) **EP 3 967 284 B1**
(45) Date of publication and mention of the grant of the patent: **02.04.2025**
(21) Application number: 20902153.4
(22) Date of filing: 16.12.2020
(51) Int. Cl.: A61F 13/49, A61F 13/56

(54) **DISPOSABLE DIAPER**
WEGWERFWINDEL
COUCHE JETABLE

(30) Priority: 16.12.2019 JP 2019226855
(43) Date of publication of application: 16.03.2022
(73) Proprietor: Koyo Corporation, Yokohama-shi, Kanagawa 236-0004 (JP)
(72) Inventor: SHIRAI, Atsuko, Yokohama-shi, Kanagawa 236-0004 (JP); MATSUMIYA, Munetada, Yokohama-shi, Kanagawa 236-0004 (JP)
(74) Representative: Samson & Partner Patentanwälte mbB
(86) International application number: PCT/JP2020/046914
(87) International publication number: WO 2021/125213

(56) References cited:
- WO-A1-2010/007872
- JP-A- 2012 176 227
- JP-A- 2018 202 023
- JP-A- H0 910 260
- JP-A- H0 910 260
- JP-A- H06 277 251
- JP-A- H06 502 091
- US-A- 5 447 508

## Description

### TECHNICAL FIELD

The present invention relates to disposable diapers, especially tape-type disposable diapers.

### BACKGROUND ART

A tape-type disposable diaper disclosed in Patent document 1 includes target tapes provided on an outer surface of a ventral member, fastening tapes that are provided so as to protrude from left and right edges of a dorsal member and can be attached to and detached from the target tapes, left and right elastic materials that are provided along left and right edges of the diaper from a part of the ventral member to the front half of the dorsal member through a middle member, and an end flap elastic material provided in a left-right direction in a region without an absorber in a center of a rear end of the dorsal member. Another tape-type disposable article is disclosed in Patent document 2.

Due to the above-mentioned arrangement of the left and right elastic materials and the end flap elastic materials, the disposable diaper is unlikely to have a shape that fits rounded buttocks of a wearer. Also, the disposable diaper is not easy to align in an appropriate position for the wearer to wear.

### RELATED ART DOCUMENTS

### PATENT DOCUMENTS

Patent document 1: Japanese Patent No.5603391
Patent document 2: JP H09 10260 A

### SUMMARY OF THE INVENTION

### PROBLEMS TO BE SOLVED BY THE INVENTION

The present invention has an object to provide a disposable diaper having a good fit to a wearer by devising an arrangement of elastic materials that expand and contract in a front-rear direction.

### MEANS OF SOLVING THE PROBLEMS

According to one aspect of the present invention, a disposable diaper includes a body that has a plurality of sheets enveloping an absorber and has a front section, a middle section, and a back section respectively corresponding to a wearer's lower abdomen, crotch, and buttocks; connecting members protruding outward from left and right side edges of the back section of the body; fastening units provided at protruding ends of the connecting members and removable to an outer surface of the body; left and right side elastic materials composed of a group of threadlike elastic materials provided from a front edge to a back edge of the body; and left and right inner elastic materials composed of a group of threadlike elastic materials provided from the front edge to the back edge of the body and being away from the left and right side elastic materials toward a center, wherein the absorber includes left and right outer bulge portions between left and right first recesses provided in a boundary area between the front section and the middle section and left and right second recesses provided in a boundary area between the middle section and the back section, wherein the group of threadlike elastic materials constituting the left and right side elastic materials are arranged diagonally in parallel from the back edge toward the left and right side edges in a back end section of the body, are arranged in parallel in the front-back direction along the left and right side edges in the back section excluding the back end section of the body, are arranged diagonally in a bundled manner in the boundary area between the back section and the middle section of the body, are arranged in parallel in the front-back direction along the left and right side edges in the middle section of the body, and are arranged diagonally in a partially bundled manner up to the front edge in the front section of the body, wherein a width between a front end of the left side elastic materials and a front end of the right side elastic materials, as well as a width between a back end of the left side elastic materials and a back end of the right side elastic materials, is narrower than a width between the left side elastic materials and the right side elastic materials in the middle section of the body, wherein the group of threadlike elastic materials constituting the left and right inner elastic materials are arranged in parallel in a curved manner so as to surround the absorber in the back section of the body, are arranged diagonally in a bundled manner toward a center in the boundary area between the back section and the middle section of the body, are arranged in parallel in the front-back direction in the middle section of the body so as to overlap on the left and right outer bulge portions of the absorber, and are arranged diagonally in a partially bundled manner up to the front edge in the front section of the body, after overlapping on the left and right side portions of the absorber, and wherein a width between a front end of the left inner elastic materials and a front end of the right inner elastic materials, as well as a width between a back end of the left inner elastic materials and a back end of the right inner elastic materials, is narrower than a width between the left inner elastic materials and the right inner elastic materials in the middle section of the body.

According to another aspect of the present invention, at least a part of the left and right inner elastic materials may intersects at the back end section of the body.

The present invention is not limited to the above object or the above aspect. Further details, features, functions, and/or effects of the present invention will be further clarified by the following description of preferred exemplary embodiments of the present invention.

### BRIEF DESCRIPTION OF THE DRAWINGS

FIG. 1 is a plan view of a disposable diaper according to a first embodiment of the present invention, which is shown in a state where elastic materials do not exert elastic force;
FIG. 2 is a cross-sectional view of the disposable diaper taken along line II-II of FIG. 1;
FIG. 3 is a front view of the disposable diaper worn;
FIG. 4 is a left rear perspective view of a disposable diaper worn; and
FIG. 5 is a plan view of a disposable diaper which is a modification of the first embodiment of the present invention, which is shown in a state where elastic materials do not exert elastic force.

### MODE FOR CARRYING OUT THE INVENTION

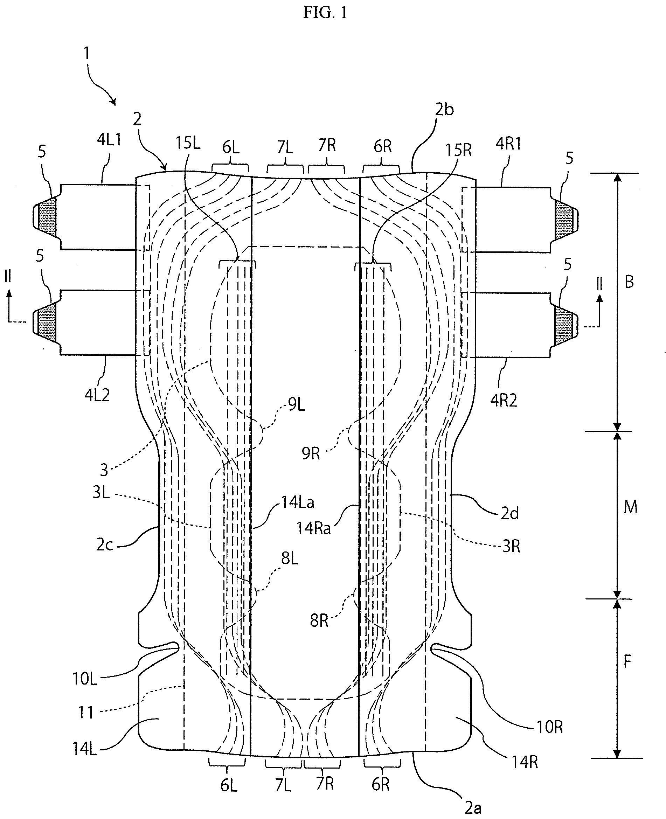

A first embodiment of the present invention will be described with reference to FIGS. 1 to 4. As shown in FIG. 1, a body 2 of a disposable diaper 1 has a front section F, a middle section M, and a back section B respectively corresponding to a lower abdomen, crotch, and buttocks of a wearer. The body 2 has a longitudinal direction in the front-back direction and a width direction in the left-right direction.

The disposable diaper 1 includes the body 2 that envelops an absorber 3 with a plurality of sheets; connecting members 4L1, 4R1, 4L2, 4R2 protruding outward from left and right side edges 2c, 2d of the back section B of the body 2; fastening unit 5 provided at each protruding end of the connecting members 4L1, 4R1, 4L2, 4R2 and removable to an outer surface of the body 2; left and right side elastic materials 6L and 6R composed of a group of threadlike elastic materials provided from a front edge 2a to a back edge 2b of the body 2; and left and right inner elastic materials 7L and 7R composed of a group of threadlike elastic materials provided from the front edge 2a to the back edge 2b of the body 2 and being away from the left and right side elastic materials 6L and 6R toward a center.

The absorber 3 has a liquid retention property and quickly absorbs a liquid such as urine discharged from the wearer and retains it inside. The raw material of the absorber 3 includes, for example, a water-absorbent fiber obtained by defibrating a roll sheet-like pulp and a powdery or granular superabsorbent polymer. For example, these raw materials are air-conveyed to a molding die installed on the peripheral surface of the suction drum and are deposited and molded in the molding die while being sucked. An absorber is manufactured by sandwiching or wrapping such a molded product with water-absorbent paper or non-woven fabric.

As shown in FIG. 1, the absorber 3 has left and right first recesses 8L and 8R toward the center in the left-right direction in the boundary area between the front section F and the middle section M of the body 2 and also has the left and right second recesses 9L and 9R toward the center in the left-right direction in the boundary area between the middle section M and the back section B. The innermost portions of the first recesses 8L and 8R of the absorber 3, as well as the innermost portions of the second recesses 9L and 9R, are closer to the center in the left-right direction than the inner ends 14La and 14Ra of left and right standing sheets 14L and 14R described later.

In the middle section M, the absorber 3 has a left outer bulge portion 3L that bulges outward greatly between the left first recess 8L and the left second recess 9L and has a right outer bulge portion 3R that bulges outward greatly between the right first recess 8R and the right second recess 9R. The disposable diaper 1 can increase the absorption capacity in the middle section M and prevent the leakage of excrement by arranging the left and right outer bulge portions 3L and 3R of the absorber 3 along thighs of the wearer.

The disposable diaper 1 improves the fit to the crotch of the wearer by appropriately matching a width between both roots of the left and right outer bulge portions 3L and 3R of the absorber 3 with a width of the crotch of the wearer. The width between both roots of the left and right outer bulge portion 3L and 3R of the absorber 3 is a distance between a line connecting both innermost portions of the left first recesses 8L and the left second recesses 9L and a line connecting both innermost portions of the right first recesses 8R and the right second recesses 9R. The width between both roots of the left and right outer bulge portion 3L and 3R of the absorber 3 is appropriately selected according to the size of the disposable diaper 1, and may be appropriately selected, for example, in a size between 80 mm and 120 mm.

The body 2 of the disposable diaper1 is an approximately rectangular shape that is long in the front-rear direction, and its width in the left-right direction in the middle section M is narrowed. The front section F of the body 2 is provided with left and right notches 10L and 10R from the left and right side edges 2c and 2d toward the left and right first recesses 8L and 8R of the absorber 3. When the middle section M of the body 2 is placed in the wearer's crotch and the left and right outer bulge portions 3L and 3R of the absorber 3 are fitted to the wearer's thigh, the front section F of the body 2 also fits the wearer's lower abdomen with the left and right notches 10L and 10R. Not limited to the above, front end corners of the front section F of the body 2 may be cut without providing the left and right notches 10L and 10R.

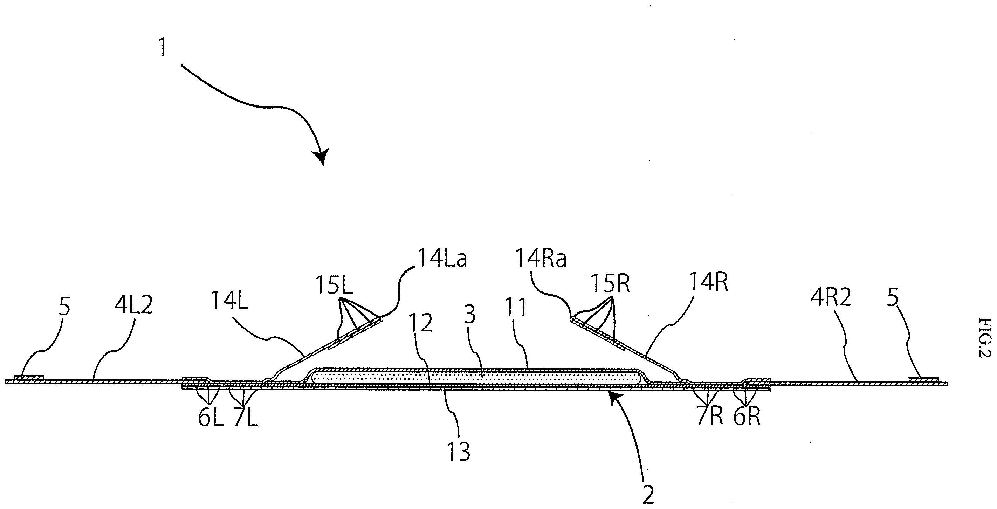

As shown in FIG. 1 or 2, the plurality of sheets of the body 2 include a top sheet 11, a bottom sheet 12, an outer sheet 13, and left and right standing sheets 14L and 14R. The length of the body 2 in the front-back direction, that is, the distance from the front edge 2a to the back edge 2b of the body 2 is the same as the length of the seats 11, 12, 13, 14L and 14R in the front-back direction. The width of the body 2 in the left-right direction, that is, the distance from the left side edge 2c to the right side edge 2d of the body 2 is the same as the width of the bottom sheet 12 and the outer sheet 13 in the left-right direction.

The top sheet 11 forms the central portion of a wearer-facing surface of the body 2. The top sheet 11 covers the entire upper surface of the absorber 3, and both are fixed with an adhesive or the like. The top sheet 11 has liquid permeability. The top sheet 11 allows a liquid such as urine discharged from the wearer to permeate and move into the absorber 3.

The top sheet 11 is made of a liquid-permeable non-woven fabric, for example, a non-woven fabric made of chemical fibers such as polyethylene, polypropylene, polyester, nylon and rayon, natural fibers such as cotton and cellulose, or a combination thereof. The top sheet 11 is not limited to the above-mentioned non-woven fabric and may be made of an appropriate material having liquid permeability, for example, a perforated film, a tissue, or a sheet obtained by appropriately joining them.

The bottom sheet 12 covers the entire lower surface of the absorber 3, and both are fixed with an adhesive or the like. The bottom sheet 12 is impermeable. The bottom sheet 12 prevents the excrement that has passed through the top sheet 11 and the excrement that has not been absorbed or retained by the absorber 3 from leaking to the outside.

The bottom sheet 12 is made of, for example, an impermeable film such as polyethylene, polypropylene, or polyester, or an impermeable non-woven fabric. The bottom sheet 12 is not limited to the above film or non-woven fabric and may be made of a suitable material having impermeable property, for example, elastic material having impermeable property, a material coated with a waterproof adhesive, a laminate thereof, or a sheet obtained by appropriately joining them.

The outer sheet 13 forms the outer surface of the body 2. The outer sheet 13 is placed under the bottom sheet 12, and both are fixed with an adhesive or the like. The outer sheet 13 is made of, for example, a chemical fiber such as polyethylene, polypropylene, polyester, nylon or rayon, a natural fiber such as cotton or cellulose, or a non-woven fabric made of a combination thereof. The outer sheet 13 may be less permeable than the bottom sheet 12.

The left and right standing sheets 14L and 14R form the left and right side portions of the wearer-facing surface of the body 2. The left and right standing sheets 14L and 14R are fixed to the front and back ends and the left edge of the top sheet 11 and the bottom sheet 12 with an adhesive or the like. Non-fixed portions of the left and right standing sheets 14L and 14R form standing units.

The left and right standing elastic materials 15L and 15R are arranged in the front-back direction along the inner end 14La and the inner end 14Ra of the left and right standing sheets 14L and 14R. The left and right standing elastic materials 15L and 15R are stretchably joined between the two layer portions of the left and right standing sheets 14L and 14R. The standing units of the left and right standing sheets 14L and 14R are gathered and rise from the top sheet 11 by contracting the left and right standing elastic materials 15L and 15R in the front-back direction. When the disposable diaper 1 is worn, the standing units of the left and right standing sheets 14L and 14R come into close contact with the wearer so as to prevent excrement from leaking to the outside.

As shown in FIG. 1 or 2, a large gap is provided between the left and right side edges of the top sheet 11 to which the left and right standing sheets 14L and 14R are fixed and the left and right side edges of the absorber 3. Thus, a large and three-dimensional excrement storage space is formed between the standing units of the left and right standing sheets 14L and 14R and the top sheet 11.

The left and right standing sheets 14L and 14R may be made of, for example, a non-woven fabric having impermeable or water-repellent properties, or may be made of the same raw material as bottom sheet 12 or outer sheet 13.

As shown in FIG. 1 or 2, connecting members 4L1, 4R1, 4L2, 4R2 protrude from the left and right side edges 2c and 2d of the back section B of the body 2. The left and right first connecting members 4L1 and 4R1 are fixed between the left and right standing sheets 14L and 14R and the bottom sheet 12 by an adhesive or heat welding near the left and right side edges 2c and 2d of the back end of the body 2. The left and right second connecting members 4L2, 4R2 are fixed at positions separated forward from the left and right first connecting members 4L1, 4R1 with an adhesive or the like.

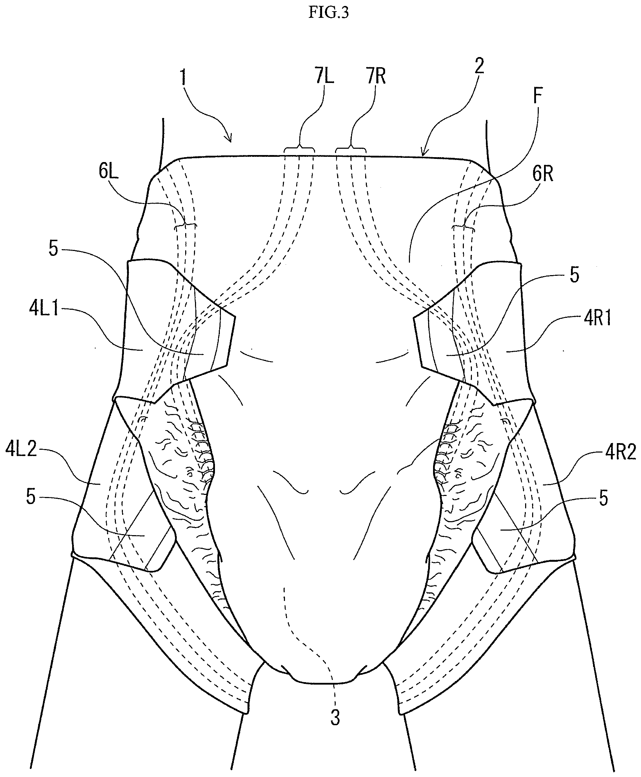

At each protruding end of the connecting members 4L1, 4R1, 4L2, 4R2, fastening unit 5 that can be attached to and detached from the outer sheet 13 forming the outer surface of the front section F of the body 2 is provided. The first connecting members 4L1, 4R1 and the second connecting members 4L2, 4R2 are separated in the front-back direction. As shown in FIG. 3, when the disposable diaper 1 is worn, it can widely adjust a direction and a position of hooking each fastening unit 5 on the outer surface of the front section F of the body 2. Thus, the disposable diaper 1 has a high fit and size compatibility to the wearer.

The connecting members 4L1, 4R1, 4L2, 4R2 are made of, for example, chemical fibers such as polyethylene, polypropylene, polyester, nylon and rayon, natural fibers such as cotton and cellulose, or a non-woven fabric made of a combination thereof.

The connecting members of the present invention are not limited to the above-mentioned non-woven fabric, and for example, a part or all of the connecting members may be made of elastic materials. When some or all of the connecting members are made of elastic materials, the front section F and the back section B of the body 2 can be appropriately adjusted and fixed by the elasticity of the connecting members 4L1, 4R1, 4L2, 4R2 when the disposable diaper1 is worn. Thus, the disposable diaper 1 has further improved fit and size compatibility to the wearer.

The fastening unit 5 may be, for example, a hook-and-loop fastener in which small pieces of hooks that can be detachably hooked on a non-woven fabric forming the outer surface of the body 2 are gathered. Further, the fastening unit 5 may be an adhesive tape having adhesiveness.

The outer surface of the outer sheet 13 or the bottom sheet 12 of the front section F of the body 2 may be printed with a mark to assist in determining a position where the fastening unit 5 is hooked. Further, a target tape having a mark for assisting the positioning of the fastening unit 5 may be fixed on the outer sheet 13 of the front section F. For example, if the fastening unit 5 is a hook-and-loop fastener, the target tape may have a loop-shaped portion that makes it easy to attach and detach the hook-and-loop fastener.

As shown in FIGS. 1 and 2, the left and right side elastic materials 6L and 6R are composed of a group of elastic materials in which a plurality of threadlike elastic materials are collected. The left and right side elastic materials 6L and 6R are stretchably joined between the outer sheet 13 and the bottom sheet 12 by adhesive or heat welding. Not limited to the above, the side elastic materials may be provided between two outer sheets and joined to them. Further, the side elastic materials may be provided between the absorber and the bottom sheet and joined to them.

As shown in FIG. 1, the left and right side elastic materials 6L and 6R are provided from the front edge 2a to the back edge 2b of the body 2. The width between a front end of the left side elastic material 6L and a front end of the right side elastic material 6R, as well as the width between a back end of the left side elastic materials 6L and a back end of the right side elastic material 6R, is narrower than the width between the left side elastic material 6L and the right side elastic material 6R in the middle section M of the body 2.

More specifically, the front and back ends of each group of threadlike elastic materials constituting the left and right side elastic materials 6L and 6R are located near each of inner edges 14La and 14Ra of the left and right standing sheets 14L and 14R at the front edge 2a and the back edge 2b of the body 2. The groups of threadlike elastic materials constituting the left and right side elastic materials 6L and 6R are arranged diagonally in parallel from the back edge 2b toward the left and right side edges 2c and 2d in the body 2's back end section where the left and right first connecting members 4L1 and 4R1 are provided, are arranged in parallel in the front-back direction along the left and right side edges 2c and 2d in the back section B excluding the back end section of the body 2, are arranged diagonally in a bundled manner in the boundary area between the back section B and the middle section M of the body 2, are arranged in parallel in the front-back direction along the left and right side edges 2c and 2d in the middle section M of the body 2, and are arranged diagonally in a partially bundled manner up to the front edge 2a in the front section F of the body 2.

As shown in FIGS. 1 and 2, the left and right inner elastic materials 7L and 7R are composed of a group of elastic materials in which a plurality of threadlike elastic materials are collected. The left and right inner elastic materials 7L and 7R are stretchably joined between the outer sheet 13 and the bottom sheet 12 by adhesive or heat welding. Not limited to the above, the inner elastic materials may be provided between the two outer sheets and joined to them. Further, the inner elastic materials may be provided between the absorber and the bottom sheet and joined to them.

As shown in FIG. 1, the left and right inner elastic materials 7L and 7R are provided from the front edge 2a to the back edge 2b of the body 2 and are separated from the left and right side elastic materials 6L and 6R toward the center in the left-right direction. The width between a back end of the left inner elastic material 7L and a back end of the right inner elastic material 7R is narrower than the width between the left inner elastic material 7L and the right inner elastic material 7R in the middle section M of the body 2. The width between a front end of the left inner elastic material 7L and a front end of the right inner elastic material 7R is narrower than the width between the left inner elastic material 7L and the right inner elastic material 7R in the middle section M of the body 2.

More specifically, the front and back ends of the group of threadlike elastic materials constituting the left and right inner elastic materials 7L and 7R are located near the center of the front edge 2a and back edge 2b of the body 2. The group of threadlike elastic materials constituting the left and right inner elastic materials 7L and 7R are arranged in parallel in a curved manner so as to surround the absorber 3 in the back section B of the body 2, are arranged diagonally in a bundled manner toward a center in the boundary area between the back section B and the middle section M of the body 2, are arranged in parallel in the front-back direction in the middle section M of the body 2 so as to overlap on the left and right outer bulge portions 3L and 3R of the absorber 3, and are arranged diagonally in a partially bundled manner up to the front edge 2a in the front section F of the body 2, after overlapping on the left and right side portions of the absorber 3.

The side elastic materials 6L and 6R, the inner elastic materials 7L and 7R, and the standing elastic materials 14L and 14R are made of stretchable materials such as natural rubber, polyurethane resin, and olefin elastomers. The standing elastic materials 14L and 14R are not limited to a plurality of threadlike elastic materials but may be one or more strip-shaped or sheet-shaped elastic materials.

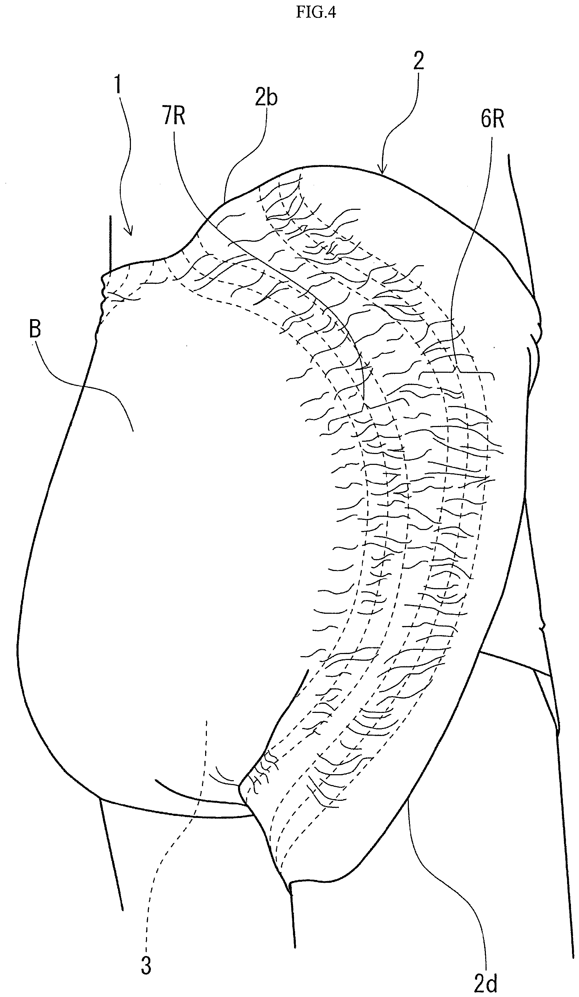

As shown in FIG. 4, when the disposable diaper 1 is worn, the part of the back section B of the body 2, where the left and right side elastic materials 6L and 6R are placed, is curved and closely attached along the circumference of the wearer's buttocks. Further, the part of the back section of the body 2, where the left and right inner elastic materials 7L and 7R are placed, is curved and closely attached along the raised buttocks of the wearer. Thus, the back section B of the body 2 can be easily and properly aligned with the wearer's buttocks, and the disposable diaper 1 improves the fit to the wearer.

As shown in FIG. 1, the group of threadlike elastic materials constituting the left and right side elastic materials 6L and 6R are arranged diagonally in parallel from the back edge 2b toward the left and right side edges 2c and 2d in the body 2's back end section where the left and right first connecting members 4L1 and 4R1 are provided. Thus, as shown in FIG. 3, when the disposable diaper 1 is worn, the first connecting members 4L1 and 4R1 are easily placed and hooked at a position above the wearer's ilium and downward diagonally on the outer surface of the front section F of the body 2.

Further, as shown in FIG. 1, the group of threadlike elastic materials constituting the left and right side elastic materials 6L and 6R, as well as the group of threadlike elastic materials constituting the left and right inner elastic materials 7L and 7R, are arranged in a bundled manner diagonally toward the center in the boundary area between the back section B and the middle section M of the body 2. Thus, the second connecting members 4L2 and 4R2 are easily placed and hooked at positions along the bases of the left and right thighs of the wearer and downward diagonally on the outer surface of the body 2. Therefore, the disposable diaper 1 can adjust the size around the waist and the size around the legs of the wearer so as to improve the fit.

Further, as shown in FIG. 1, in the middle section M of the body 2 of the disposable diaper 1, the left and right outer bulge portions 3L and 3R of the absorber 3 are arranged along the wearer's thigh, and the group of threadlike elastic materials constituting the left and right inner elastic materials 7L and 7R are arranged in parallel in the front-back direction so as to overlap on the left and right outer bulge portions 3L and 3R. Thus, the left and right outer bulge portions 3L and 3R are supported by the left and right inner elastic materials 7L and 7R and can increase the absorption capacity in the middle section M so as to prevent the leakage of excrement.

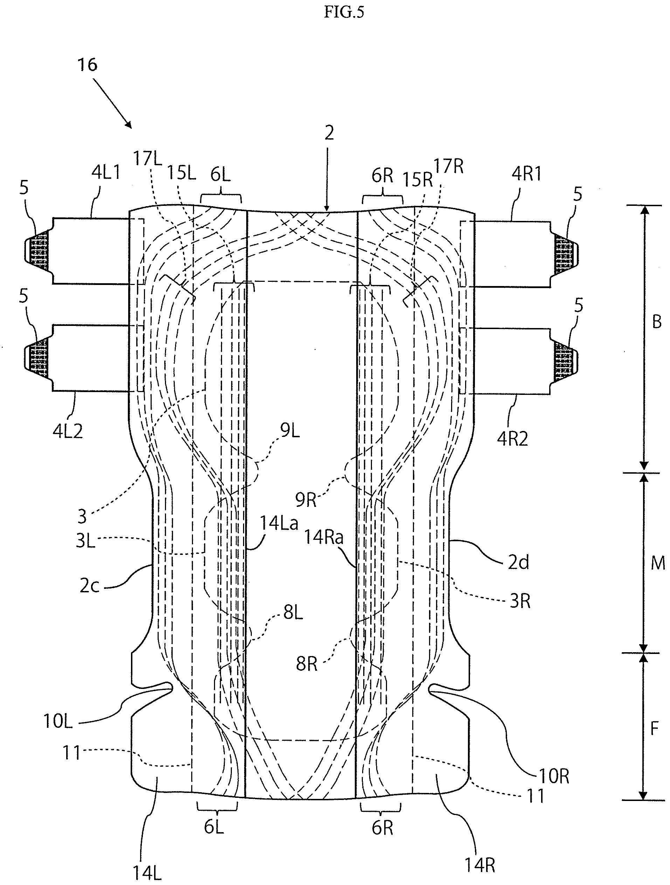

Next, as shown in FIG. 5, a disposable diaper 16 is a modification of the disposable diaper 1 of the first embodiment of the present invention. In the back section B of the body 2 of the disposable diaper16, all of the left and right inner elastic materials 17L and 17R intersect. Further, in the front section F of the body 2, some of the left and right inner elastic materials 17L and 17R intersect. Since the left and right inner elastic materials 17L and 17R intersect in the back section B of the body 2 so as to increase their length that extends diagonally, the diaper increases the contraction force around the waist of the wearer and improves the fits to the wearer.

The disposable diaper of the present invention is not limited to the above-described embodiment and includes, for example, a modified embodiment understood from the above-described embodiment and a modified embodiment that does not change the essence of the present invention.

For example, the present invention and the disposable diapers 1 and 16 of the above-described embodiment may not include the left and right standing sheets and the standing elastic materials but may instead include the left and right outer sheets. Such disposable diapers are suitable, for example, for use with an absorbent pad with an upright gather, for use by a wearer with low urine output, or for simple construction and low cost production.

Further, the present invention and the disposable diapers of the above-described embodiment may be in a state where the left and right side elastic materials and/or the left and right inner elastic materials do not exert the elastic force at the front section and/or the back end section of the body. Thus, the diaper prevents an opening around the waist from shrinking, does not spoil its appearance, and is easy to put on. In order to prevent a desired part of the elastic materials from exerting elastic force, for example, the part of the elastic materials may not be joined to the body, may be finely broken with a cutter, or may be finely heat-fused.

Further, the plurality of sheets of the body of the disposable diaper of the present invention are not limited to those of the above-described embodiment. For example, the width of the top sheet in the left-right direction may be the same as the width of the body in the left-right direction, and the left and right standing sheets or the left and right outer sheets may be provided on the left and right sides of the top sheet and fixed with an adhesive or the like. Further, the plurality of sheets of another embodiment may have different lengths in the front-rear direction or different widths in the left-right direction. In particular, the left-right width of the bottom sheet may be smaller than the left-right width of the outer sheet.

Further, the present invention and the disposable diapers of the above-described embodiment may use a bottom sheet as an outer sheet without providing an outer sheet. A suitable bottom sheet in this case may be made of a non-woven fabric or the like which has impermeable liquid property and can be hooked by fastening units. Further, the outer surface of the bottom sheet may be printed with a mark that assists in determining the hooking position of the fastening units or may be fixed with a target tape on which a mark for assisting the positioning of the fastening units is displayed.

Further, the outer surface of the body of the disposable diaper of the present invention may be a plurality of layered non-woven fabric sheets. For example, two outer sheets made of non-woven fabric may be overlapped and fixed to each other with an adhesive. The side elastic materials and/or the inner elastic materials may be provided between the two outer sheets. When the two outer sheets made of non-woven fabric are overlapped, the outer surface of the body becomes uneven so as to be easily hooked by the fastening units. Thus, it is not necessary to provide the loop-shaped portion on the attachable and removable surface of the fastening units, so that the manufacturing cost and labor can be reduced.

### INDUSTRIAL APPLICABILITY

The present invention can be used to provide a tape-type disposable diaper having excellent fit, and in particular, to provide a tape-type disposable diaper that is continuously manufactured industrially.

### DESCRIPTION OF THE REFERENCE NUMERAL

1 and 16: disposable diaper,
2: body,
3: absorber,
3L and 3R: outer bulge portion,
4L1, 4R1, 4L2, and 4R2: connecting member,
5: fastening unit,
6L and 6R: side elastic material,
7L, 7R, 17L, and 17R: inner elastic material,
8L and 8R: first recess,
9L and 9R: second recess,
10L and 10R: notch,
11: top sheet,
12: bottom sheet,
13: outer sheet,
14L and 14R: standing sheet,
15L and 15R: standing elastic material,
F: front section,
M: middle section, and
B: back section.

## Claims

1. A disposable diaper (1, 16) comprising:
a body that has a plurality of sheets (11, 12, 13, 14L, 14R) enveloping an absorber (3) and has a front section (F), a middle section (M), and a back section (B) respectively corresponding to a wearer's lower abdomen, crotch, and buttocks;
connecting members (4L1, 4R1, 4L2, 4R2) protruding outward from left and right side edges (2c, 2d) of the back section (B) of the body (2);
fastening units (5) provided at protruding ends of the connecting members (4L1, 4R1, 4L2, 4R2) and removable to an outer surface of the body (2);
left and right side elastic materials (6L, 6R) composed of a group of threadlike elastic materials provided from a front edge (2a) to a back edge (2b) of the body (2); and
left and right inner elastic materials (7L, 7R) composed of a group of threadlike elastic materials provided from the front edge (2a) to the back edge (2b) of the body (2) and being away from the left and right side elastic materials (6L, 6R) toward a center,
wherein the absorber (3) comprises left and right outer bulge portions (3L, 3R) between left and right first recesses (8L, 8R) provided in a boundary area between the front section (F) and the middle section (M) and left and right second recesses (9L, 9R) provided in a boundary area between the middle section (M) and the back section (B),
wherein the group of threadlike elastic materials constituting the left and right side elastic materials (6L, 6R) are arranged diagonally in parallel from the back edge (2b) toward the left and right side edges (2c, 2d) in a back end section of the body (2), are arranged in parallel in the front-back direction along the left and right side edges (2c, 2d) in the back section (B) excluding the back end section of the body (2), are arranged diagonally in a bundled manner in the boundary area between the back section (B) and the middle section (M) of the body (2), are arranged in parallel in the front-back direction along the left and right side edges (2c, 2d) in the middle section (M) of the body (2), and are arranged diagonally in a partially bundled manner up to the front edge (2a) in the front section (F) of the body (2),
wherein a width between a front end of the left side elastic materials (6L) and a front end of the right side elastic materials (6R), as well as a width between a back end of the left side elastic materials (6L) and a back end of the right side elastic materials (6R), is narrower than a width between the left side elastic materials (6L) and the right side elastic materials (6R) in the middle section (M) of the body (2),
wherein the group of threadlike elastic materials constituting the left and right inner elastic materials (7L, 7R) are arranged in parallel in a curved manner so as to surround the absorber (3) in the back section (B) of the body (2), are arranged diagonally in a bundled manner toward a center in the boundary area between the back section (B) and the middle section (M) of the body (2), are arranged in parallel in the front-back direction in the middle section (M) of the body (2) so as to overlap on the left and right outer bulge portions (3L, 3R) of the absorber (3), and are arranged diagonally in a partially bundled manner up to the front edge (2a) in the front section (F) of the body (2), after overlapping on the left and right side portions of the absorber (3), and
wherein a width between a front end of the left inner elastic materials (7L) and a front end of the right inner elastic materials (7R), as well as a width between a back end of the left inner elastic materials (7L) and a back end of the right inner elastic materials (7R), is narrower than a width between the left inner elastic materials (7L) and the right inner elastic materials (7R) in the middle section (M) of the body (2).

2. The disposable diaper (16) according to claim 1,
wherein at least a part of the left and right inner elastic materials (17L, 17R) intersects at the back end section of the body (2).

## Patentansprüche

1. Einwegwindel (1, 16), umfassend:
einen Körper, der eine Vielzahl von Lagen (11, 12, 13, 14L, 14R) aufweist, die einen Absorber (3) umhüllen, und einen vorderen Abschnitt (F), einen mittleren Abschnitt (M) und einen hinteren Abschnitt (B) aufweist, die jeweils dem Unterbauch, dem Schritt und dem Gesäß eines Trägers entsprechen;
Verbindungselemente (4L1, 4R1, 4L2, 4R2), die von linken und rechten Seitenrändern (2c, 2d) des hinteren Abschnitts (B) des Körpers (2) nach außen ragen;
Befestigungseinheiten (5), die an vorstehenden Enden der Verbindungselemente (4L1, 4R1, 4L2, 4R2) vorgesehen und an einer Außenfläche des Körpers (2) abnehmbar sind;
linke und rechte seitliche elastische Materialien (6L, 6R), die aus einer Gruppe fadenförmiger elastischer Materialien bestehen, die von einem vorderen Rand (2a) zu einem hinteren Rand (2b) des Körpers (2) vorgesehen sind; und
linke und rechte innere elastische Materialien (7L, 7R), die aus einer Gruppe fadenförmiger elastischer Materialien bestehen, die vom vorderen Rand (2a) zum hinteren Rand (2b) des Körpers (2) vorgesehen sind und von den linken und rechten seitlichen elastischen Materialien (6L, 6R) in Richtung einer Mitte entfernt sind,
wobei der Absorber (3) linke und rechte äußere Wölbungsabschnitte (3L, 3R) zwischen linken und rechten ersten Ausnehmungen (8L, 8R), die in einem Grenzbereich zwischen dem vorderen Abschnitt (F) und dem mittleren Abschnitt (M) vorgesehen sind, und linken und rechten zweiten Ausnehmungen (9L, 9R), die in einem Grenzbereich zwischen dem mittleren Abschnitt (M) und dem hinteren Abschnitt (B) vorgesehen sind, umfasst,
wobei die Gruppe der fadenförmigen elastischen Materialien, die die linken und rechten seitlichen elastischen Materialien (6L, 6R) bilden, diagonal parallel von dem hinteren Rand (2b) zu den linken und rechten Seitenrändern (2c, 2d) in einem hinteren Endabschnitt des Körpers (2) angeordnet sind, parallel in der Vorderseiten-Rückseiten-Richtung entlang der linken und rechten Seitenrändern (2c, 2d) in dem hinteren Abschnitt (B) mit Ausnahme des hinteren Endabschnitts des Körpers (2) angeordnet sind, im Grenzbereich zwischen dem hinteren Abschnitt (B) und dem mittleren Abschnitt (M) des Körpers (2) diagonal gebündelt angeordnet sind, im mittleren Abschnitt (M) des Körpers (2) entlang der linken und rechten Seitenränder (2c, 2d) in Vorderseiten-Rückseiten-Richtung parallel angeordnet sind und im vorderen Abschnitt (F) des Körpers (2) diagonal teilweise gebündelt bis zum vorderen Rand (2a) angeordnet sind,
wobei eine Breite zwischen einem vorderen Ende der linksseitigen elastischen Materialien (6L) und einem vorderen Ende der rechtsseitigen elastischen Materialien (6R) sowie eine Breite zwischen einem hinteren Ende der linksseitigen elastischen Materialien (6L) und einem hinteren Ende der rechtsseitigen elastischen Materialien (6R) schmaler ist als eine Breite zwischen den linksseitigen elastischen Materialien (6L) und den rechtsseitigen elastischen Materialien (6R) im mittleren Abschnitt (M) des Körpers (2),
wobei die Gruppe fadenförmiger elastischer Materialien, die die linken und rechten inneren elastischen Materialien (7L, 7R) bilden, parallel in einer gekrümmten Weise angeordnet sind, um den Absorber (3) im hinteren Abschnitt (B) des Körpers (2) zu umgeben, diagonal in einer gebündelten Weise zu einer Mitte im Grenzbereich zwischen dem hinteren Abschnitt (B) und dem mittleren Abschnitt (M) des Körpers (2) angeordnet sind, in dem mittleren Abschnitt (M) des Körpers (2) parallel in der Vorderseiten-Rückseiten-Richtung angeordnet sind, so dass sie sich an den linken und rechten äußeren Wölbungsabschnitten (3L, 3R) des Absorbers (3) überlappen, und diagonal in einer teilweise gebündelten Weise bis zu dem vorderen Rand (2a) in dem vorderen Abschnitt (F) des Körpers (2) angeordnet sind, nachdem sie sich an den linken und rechten Seitenabschnitten des Absorbers (3) überlappen, und
wobei eine Breite zwischen einem vorderen Ende der linken inneren elastischen Materialien (7L) und einem vorderen Ende der rechten inneren elastischen Materialien (7R) sowie eine Breite zwischen einem hinteren Ende der linken inneren elastischen Materialien (7L) und einem hinteren Ende der rechten inneren elastischen Materialien (7R) schmaler ist als eine Breite zwischen den linken inneren elastischen Materialien (7L) und den rechten inneren elastischen Materialien (7R) in dem mittleren Abschnitt (M) des Körpers (2).

2. Einwegwindel (16) nach Anspruch 1,
wobei sich zumindest ein Teil des linken und rechten inneren elastischen Materials (17L, 17R) am hinteren Endabschnitt des Körpers (2) überschneidet.

## Revendications

1. Couche jetable (1, 16) comprenant :
un corps qui présente une pluralité de feuilles (11, 12, 13, 14L, 14R) enveloppant un absorbeur (3) et présente une section avant (F), une section centrale (M), et une section arrière (B) correspondant respectivement à la partie inférieure de l'abdomen, à l'entrejambe, et aux fesses d'un porteur ;
des éléments de liaison (4L1, 4R1, 4L2, 4R2) faisant saillie vers l'extérieur à partir des bords latéraux gauche et droit (2c, 2d) de la section arrière (B) du corps (2) ;
des unités de fixation (5) prévues au niveau d'extrémités en saillie des éléments de liaison (4L1, 4R1, 4L2, 4R2) et de manière amovible par rapport à une surface extérieure du corps (2) ;
des matériaux élastiques latéraux gauches et droits (6L, 6R) composés d'un groupe de matériaux élastiques filiformes prévus à partir d'un bord avant (2a) vers un bord arrière (2b) du corps (2) ; et
des matériaux élastiques intérieurs gauches et droits (7L, 7R) composés d'un groupe de matériaux élastiques filiformes prévus à partir du bord avant (2a) vers le bord arrière (2b) du corps (2) et éloignés des matériaux élastiques latéraux gauches et droits (6L, 6R) en direction d'un centre,
dans laquelle l'absorbeur (3) comprend des parties de renflement extérieures gauche et droite (3L, 3R) entre des premiers évidements gauche et droit (8L, 8R) prévus dans une zone limite entre la section avant (F) et la section centrale (M) et des deuxièmes évidements gauche et droit (9L, 9R) prévus dans une zone limite entre la section centrale (M) et la section arrière (B),
dans laquelle le groupe de matériaux élastiques filiformes constituant les matériaux élastiques latéraux gauches et droits (6L, 6R) sont agencés en diagonale en parallèle à partir du bord arrière (2b) en direction des bords latéraux gauche et droit (2c, 2d) dans une section d'extrémité arrière du corps (2), sont agencés en parallèle dans la direction avant-arrière le long des bords latéraux gauche et droit (2c, 2d) dans la section arrière (B) à l'exclusion de la section d'extrémité arrière du corps (2), sont agencés en diagonale d'une manière regroupée dans la zone limite entre la section arrière (B) et la section centrale (M) du corps (2), sont agencés en parallèle dans la direction avant-arrière le long des bords latéraux gauche et droit (2c, 2d) dans la section centrale (M) du corps (2), et sont agencés en diagonale d'une manière en partie regroupée jusqu'au bord avant (2a) dans la section avant (F) du corps (2),
dans laquelle une largeur entre une extrémité avant des matériaux élastiques latéraux gauches (6L) et une extrémité avant des matériaux élastiques latéraux droits (6R), ainsi qu'une largeur entre une extrémité arrière des matériaux élastiques latéraux gauches (6L) et une extrémité arrière des matériaux élastiques latéraux droits (6R), est plus étroite qu'une largeur entre les matériaux élastiques latéraux gauches (6L) et les matériaux élastiques latéraux droits (6R) dans la section centrale (M) du corps (2),
dans laquelle le groupe de matériaux élastiques filiformes constituant les matériaux élastiques intérieurs gauches et droits (7L, 7R) sont agencés en parallèle d'une manière courbée de façon à entourer l'absorbeur (3) dans la section arrière (B) du corps (2), sont agencés en diagonale d'une manière regroupée en direction d'un centre dans la zone limite entre la section arrière (B) et la section centrale (M) du corps (2), sont agencés en parallèle dans la direction avant-arrière dans la section centrale (M) du corps (2) de façon à chevaucher les parties de renflement extérieures gauche et droite (3L, 3R) de l'absorbeur (3), et sont agencés en diagonale d'une manière en partie regroupée jusqu'au bord avant (2a) dans la section avant (F) du corps (2), après chevauchement des parties latérales gauche et droite de l'absorbeur (3), et
dans laquelle une largeur entre une extrémité avant des matériaux élastiques intérieurs gauches (7L) et une extrémité avant des matériaux élastiques intérieurs droits (7R), ainsi qu'une largeur entre une extrémité arrière des matériaux élastiques intérieurs gauches (7L) et une extrémité arrière des matériaux élastiques intérieurs droits (7R), est plus étroite qu'une largeur entre les matériaux élastiques intérieurs gauches (7L) et les matériaux élastiques intérieurs droits (7R) dans la section centrale (M) du corps (2).

2. Couche jetable (16) selon la revendication 1,
dans laquelle au moins une partie des matériaux élastiques intérieurs gauches et droits (17L, 17R) se croise au niveau de la section d'extrémité arrière du corps (2).
